# EUROPEAN PATENT APPLICATION

(11) **EP 4 706 397 A1**
(43) Date of publication of application: **11.03.2026**
(21) Application number: 24780762.1
(22) Date of filing: 29.03.2024
(51) Int. Cl.: A23C 9/123, A23C 9/13, A23L 2/38, A23L 2/44, A23L 2/52, A23L 5/00, A23L 33/135

(54) **FOOD PRODUCT INCLUDING VIABLE CELLS OF LACTIC ACID BACTERIA, METHOD FOR PRODUCING SAME, AND METHOD FOR INHIBITING INCREASE IN ACIDITY AND/OR DECREASE IN NUMBER OF VIABLE CELLS**

(30) Priority: 31.03.2023 JP 2023056874; 31.10.2023 JP 2023186283
(71) Applicant: MITSUBISHI CHEMICAL CORPORATION, Tokyo 100-8251 (JP)
(72) Inventor: SAKURADA Nobuyuki, Tokyo 100-8251 (JP); KITAYAMA Sachiko, Tokyo 100-8251 (JP); MATSUURA Tsutashi, Tokyo 100-8251 (JP); HABU Masaya, Tokyo 100-8251 (JP)
(74) Representative: Hoffmann Eitle
(86) International application number: PCT/JP2024/012946
(87) International publication number: WO 2024/204667

(57) **Abstract**

As a technique for suppressing increase in acidity and decrease in the number of viable bacteria during storage of a food product containing a viable lactic acid bacterium, and containing a small amount of a nonfat milk solid content or protein serving as nutrients for lactic acid bacteria, such as a lactic acid bacteria beverage and a fermented milk-based lactic acid bacteria beverage, the present invention provides a food product comprising a milk ingredient and a viable lactic acid bacterium, having a nonfat milk solid content of less than 8.0 wt%, a protein content of less than 2.7 wt%, and a pH of 4.6 or less, the food product comprising an emulsifier and a water activity reducing agent.

## Description

### Technical Field

The present disclosure relates to a food product containing a viable lactic acid bacterium, a method for producing the same, and a method for suppressing increase in acidity and/or decrease in the number of viable bacteria. More specifically, the present disclosure relates to a food product containing a viable lactic acid bacterium having low nonfat milk solid content or protein content in which increase in acidity and decrease in the number of viable bacteria during storage are suppressed, and the like.

### Background Art

A problem of a food product containing a viable lactic acid bacterium is that during the period of storage from production through distribution to be eaten by consumers, the acidity increases due to lactic acid bacterial metabolism, which causes decrease in the number of viable lactic acid bacteria and flavor deterioration.

For fermented milk (yogurt), various technologies have been suggested to suppress the increase in acidity and the decrease in the number of viable bacteria during storage.

For example, Patent Literature 1 discloses, regarding low-fat fermented food products obtained by culturing bacteria of the genus *Bifidobacterium* in a low-fat medium, a "method for producing a low-fat fermented food, comprising inoculating a bacterium of the genus *Bifidobacterium* into a medium with a fat content of 2.5 mass% or less, culturing the resultant to obtain a fermented liquid, and mixing the fermented liquid with a saturated fatty acid, a salt thereof, or an ester thereof" (see Claim 9) as a technique for maintaining high the number of viable bacteria even after storage.

Patent Literature 2 describes, as a technique for suppressing increase in acidity during storage and improving the flavor and texture of fermented milk, a technique of fermenting a raw material composition to obtain fermented milk, and then adding a fatty acid ester thereto as a food emulsifier.

Neither Patent Literature 1 nor Patent Literature 2 describes combined use of an emulsifier and a water activity reducing agent.

Lactic acid bacteria beverages and fermented milk-based lactic acid bacteria beverages preferably have a lower nonfat milk solid content so that a more refreshing flavor can be provided and lactic acid bacteria can be more simply eaten compared to fermented milk. It is known that maintaining the number of viable lactic acid bacteria is even more difficult in lactic acid bacteria beverages and fermented milk-based lactic acid bacteria beverages (Non Patent Literature 1). This is because, compared to fermented milk containing a large amount of nonfat milk solid contents, namely, proteins and carbohydrates that serve as nutrients for lactic acid bacteria, lactic acid bacteria beverages and fermented milk-based lactic acid bacteria beverages are diluted with water or the like for ensuring fluidity, which results in a nutrient-poor environment having a lower nonfat milk solid content. Therefore, to suppress the increase in acidity and the decrease in the number of viable bacteria during storage of beverages, approaches, different from those for fermented milk, have been proposed for lactic acid bacteria beverages and fermented milk-based lactic acid bacteria beverages.

For example, as a technique for maintaining the number of viable bacteria in lactic acid bacteria beverages, Patent Literature 3 proposes optimizing a sugar content (Brix) in a concentrated auxiliary raw material in mixing the auxiliary raw material with a lactic acid bacterium. According to this technique, by suppressing the Brix (sugar content) in the auxiliary material to 28% or less in mixing lactic acid bacteria with a sugar solution in a fermented product, stress on the lactic acid bacteria is reduced, and thus, viability of the lactic acid bacteria during subsequent processing and storage is kept high.

### Citation List

### Patent Literature

Patent Literature 1: JP 2020-162478 A
Patent Literature 2: WO2021/215530
Patent Literature 3: JP 2022-143723 A

### Non Patent Literature

Non Patent Literature 1: Nyusankin no Hatsuiku ni Oyobosu Nyukokeibun Nodo no Eikyo (Influence of Milk Solid Concentration on Lactic Acid Bacteria Growth), Yano et al., Nichiku Kaiho (Journal the Japanese Society of Zootechnical Science), 31 (4), 204 - 208, 1960

### Summary of Invention

### Technical Problem

A principal object of the present disclosure is to provide a technique for suppressing increase in acidity and decrease in the number of viable bacteria during storage in a food product containing a viable lactic acid bacterium, and containing a small amount of a nonfat milk solid content or protein serving as nutrients for lactic acid bacteria, such as a lactic acid bacteria beverage and a fermented milk-based lactic acid bacteria beverage.

### Solution to Problem

For solving the above-described problem, the present disclosure provides the following [1] to [44]:
[1] A food product comprising a milk ingredient and a viable lactic acid bacterium, having a nonfat milk solid content of less than 8.0 wt%, a protein content of less than 2.7 wt%, and a pH of 4.6 or less, the food product comprising an emulsifier and a water activity reducing agent.
   [1-2] The food product according to [1], wherein a content of fats or oils is less than 10 wt%.
   [1-3] The food product according to [1] or [2], wherein the emulsifier has an HLB (Hydrophilic-Lipophilic Balance) of 9 or more.
   [1-4] The food product according to any one of [1] to [1-3], wherein a content of the water activity reducing agent is 5 wt% or more.
[2] The food product according to any one of [1] to [1-4], wherein the emulsifier is one or more fatty acid esters selected from the group consisting of a sucrose fatty acid ester, a monoglyceride, an organic acid monoglyceride, a polyglycerol fatty acid ester, and a stearoyl lactylate, and is preferably a sucrose monofatty acid ester and/or a stearoyl lactylate.
[3] The food product according to [2], wherein the emulsifier is a sucrose monofatty acid ester and/or a stearoyl lactylate, and is contained in an amount of 0.001 to 1.0 wt%.
[4] The food product according to any one of [1] to [3], wherein the water activity reducing agent is one or more selected from the group consisting of glucose, fructose, sucrose, erythritol, sorbitol, mannitol, isomaltooligosaccharide, xylooligosaccharide, oligotose, maltitol, palatinose, and xylitol, and is preferably glucose, fructose, and/or sucrose.
[5] The food product according to any one of [1] to [4], having water activity Aw at 25°C of 0.990 or less, and preferably 0.981 or less.
[6] The food product according to any one of [1] to [5], comprising the viable lactic acid bacterium in an amount of 1 × 10² cfu/ml or more.
[7] The food product according to any one of [1] to [6], having a Y₁/X₁ value, calculated from an acidity increase rate X₁ (%) and a bacterial survival rate Y₁ (%) after storage at 25°C for 21 days from production of 0.012 or more.
[8] The food product according to any one of [1] to [7], having a Y₂/X₂ value, calculated from an acidity increase rate X₂ (%) and a bacterial survival rate Y₂ (%) after storage at 30°C for 14 days from production of 0.012 or more.
[9] The food product according to any one of [1] to [8], wherein the lactic acid bacterium is of the *Lactobacillus* genus *(Lactocaseibacillus* genus).
[10] A method for producing a food product comprising a milk ingredient and a viable lactic acid bacterium, having a nonfat milk solid content of less than 8.0 wt%, a protein content of less than 2.7 wt%, and a pH of 4.6 or less, the method comprising: (1) a step of adding a water activity reducing agent to the milk ingredient before fermentation by the lactic acid bacterium, and a step of adding an emulsifier to the milk ingredient after fermentation by the lactic acid bacterium; (2) a step of adding a water activity reducing agent to the milk ingredient before fermentation by the lactic acid bacterium, and a step of adding an emulsifier and a water activity reducing agent to the milk ingredient after fermentation by the lactic acid bacterium; or (3) a step of adding an emulsifier and a water activity reducing agent to the milk ingredient after fermentation by the lactic acid bacterium.
   [10-2] The production method according to [10], wherein a content of fats or oils in the food product is less than 10 wt%.
   [10-3] The production method according to [10] or [10-2], wherein the emulsifier has an HLB of 9 or more.
   [10-4] The production method according to any one of [10] to [10-3], wherein a content of the water activity reducing agent in the food product is 5 wt% or more.
[11] The production method according to any one of [10] to [10-4], wherein the emulsifier is one or more fatty acid esters selected from the group consisting of a sucrose fatty acid ester, a monoglyceride, an organic acid monoglyceride, a polyglycerol fatty acid ester, and a stearoyl lactylate, and is preferably a sucrose monofatty acid ester and/or a stearoyl lactylate.
[12] The production method according to [11], wherein a sucrose monofatty acid ester and/or a stearoyl lactylate is added, as the emulsifier, in an amount of 0.001 to 1.0 wt%.
[13] The production method according to any one of [10] to [12], wherein the water activity reducing agent is one or more selected from the group consisting of glucose, fructose, sucrose, erythritol, sorbitol, mannitol, isomaltooligosaccharide, xylooligosaccharide, oligotose, maltitol, palatinose, and xylitol, and is preferably glucose, fructose, and/or sucrose.
[14] The production method according to any one of [10] to [13], wherein the food product has water activity Aw at 25°C of 0.990 or less, and preferably 0.981 or less.
[15] The production method according to any one of [10] to [14], wherein the food product comprises the viable lactic acid bacterium in an amount of 1 × 10² cfu/ml or more.
[16] The production method according to any one of [10] to [15], wherein the lactic acid bacterium is of the *Lactobacillus* genus (*Lactocaseibacillus* genus).
[17] A method for suppressing increase in acidity and/or decrease in the number of viable bacteria, after production, in a food product comprising a milk ingredient and a viable lactic acid bacterium, having a nonfat milk solid content of less than 8.0 wt%, a protein content of less than 2.7 wt%, and a pH of 4.6 or less, the method comprising: (1) a step of adding a water activity reducing agent to the milk ingredient before fermentation by the lactic acid bacterium, and a step of adding an emulsifier to the milk ingredient after fermentation by the lactic acid bacterium; (2) a step of adding a water activity reducing agent to the milk ingredient before fermentation by the lactic acid bacterium, and a step of adding an emulsifier and a water activity reducing agent to the milk ingredient after fermentation by the lactic acid bacterium; or (3) a step of adding an emulsifier and a water activity reducing agent to the milk ingredient after fermentation by the lactic acid bacterium.
   [17-2] The method according to [17], wherein a content of fats or oils in the food product is less than 10 wt%.
   [17-3] The method according to [17] or [17-2], wherein the emulsifier has an HLB of 9 or more.
   [17-4] The method according to any one of [17] to [17-3], wherein a content of the water activity reducing agent in the food product is 5 wt% or more.
[18] The method according to any one of [17] to [17-4], wherein the emulsifier is one or more fatty acid esters selected from the group consisting of a sucrose fatty acid ester, a monoglyceride, an organic acid monoglyceride, a polyglycerol fatty acid ester, and a stearoyl lactylate, and is preferably a sucrose monofatty acid ester and/or a stearoyl lactylate.
[19] The method according to [18], wherein a sucrose monofatty acid ester and/or a stearoyl lactylate is added, as the emulsifier, in an amount of 0.001 to 1.0 wt%.
[20] The method according to any one of [17] to [19], wherein the water activity reducing agent is one or more selected from the group consisting of glucose, fructose, sucrose, erythritol, sorbitol, mannitol, isomaltooligosaccharide, xylooligosaccharide, oligotose, maltitol, palatinose, and xylitol, and is preferably glucose, fructose, and/or sucrose.
[21] The method according to any one of [17] to [20], wherein the food product has water activity Aw at 25°C of 0.990 or less, and preferably 0.981 or less.
[22] The method according to any one of [17] to [21], wherein the food product comprises the viable lactic acid bacterium in an amount of 1 × 10² cfu/ml or more.
[23] The method according to any one of [17] to [22], wherein a Y₁/X₁ value, calculated from an acidity increase rate X₁ (%) and a bacterial survival rate Y₁ (%) after storage at 25°C for 21 days from production of the food product is 0.012 or more.
[24] The method according to any one of [17] to [23], wherein a Y₂/X₂ value, calculated from an acidity increase rate X₂ (%) and a bacterial survival rate Y₂ (%) after storage at 30°C for 14 days from production of the food product is 0.012 or more.
[25] The method according to any one of [17] to [24], wherein the lactic acid bacterium is of the *Lactobacillus* genus (*Lactocaseibacillus* genus).
[26] Use of an emulsifier and a water activity reducing agent for suppressing increase in acidity and/or decrease in the number of viable bacteria, after production, in a food product comprising a milk ingredient and a viable lactic acid bacterium, having a nonfat milk solid content of less than 8.0 wt%, a protein content of less than 2.7 wt%, and a pH of 4.6 or less.
   [26-2] The use according to [26], wherein a content of fats or oils in the food product is less than 10 wt%.
   [26-3] The use according to [26] or [26-2], wherein the emulsifier has an HLB of 9 or more.
   [26-4] The use according to any one of [26] to [26-3], wherein a content of the water activity reducing agent in the food product is 5 wt% or more.
[27] The use according to any one of [26] to [26-4], wherein the emulsifier is one or more fatty acid esters selected from the group consisting of a sucrose fatty acid ester, a monoglyceride, an organic acid monoglyceride, a polyglycerol fatty acid ester, and a stearoyl lactylate, and is preferably a sucrose monofatty acid ester and/or a stearoyl lactylate.
[28] The use according to [27], wherein a sucrose monofatty acid ester and/or a stearoyl lactylate is added, as the emulsifier, in an amount of 0.001 to 1.0 wt%.
[29] The use according to any one of [26] to [28], wherein the water activity reducing agent is one or more selected from the group consisting of glucose, fructose, sucrose, erythritol, sorbitol, mannitol, isomaltooligosaccharide, xylooligosaccharide, oligotose, maltitol, palatinose, and xylitol, and is preferably glucose, fructose, and/or sucrose.
[30] The use according to any one of [26] to [29], wherein the food product has water activity Aw at 25°C of 0.990 or less, and preferably 0.981 or less.
[31] The use according to any one of [26] to [30], wherein the food product comprises the viable lactic acid bacterium in an amount of 1 × 10² cfu/ml or more.
[32] The use according to any one of [26] to [31], wherein a Y₁/X₁ value, calculated from an acidity increase rate X₁ (%) and a bacterial survival rate Y₁ (%) after storage at 25°C for 21 days from production of the food product is 0.012 or more.
[33] The use according to any one of [26] to [32], wherein a Y₂/X₂ value, calculated from an acidity increase rate X₂ (%) and a bacterial survival rate Y₂ (%) after storage at 30°C for 14 days from production of the food product is 0.012 or more.
[34] The use according to any one of [26] to [33], wherein the lactic acid bacterium is of the *Lactobacillus* genus (*Lactocaseibacillus* genus).
[35] A food product comprising a milk ingredient and a viable lactic acid bacterium, having a nonfat milk solid content of less than 8.0 wt%, a protein content of less than 2.7 wt%, and a content of fats or oils of less than 10 wt%, the food product comprising an emulsifier having an HLB of 9 or more, and having water activity Aw at 25°C of 0.990 or less, and preferably 0.981 or less.
[36] The food product according to [35], further comprising a water activity reducing agent.
[37] The food product according to [35] or [36], wherein a content of the water activity reducing agent is 5 wt% or more.
[38] The food product according to any one of [35] to [37], having a pH of 4.6 or more.
[39] The food product according to any one of [35] to [38], wherein the emulsifier is one or more fatty acid esters selected from the group consisting of a sucrose fatty acid ester, a monoglyceride, an organic acid monoglyceride, a polyglycerol fatty acid ester, and a stearoyl lactylate, and is preferably a sucrose monofatty acid ester and/or stearoyl lactylate.
[40] The food product according to [39], wherein the emulsifier is a sucrose monofatty acid ester and/or a stearoyl lactylate, and contained in an amount of 0.001 to 1.0 wt%.
[41] The food product according to any one of [36] to [40], wherein the water activity reducing agent is one or more selected from the group consisting of glucose, fructose, sucrose, erythritol, sorbitol, mannitol, isomaltooligosaccharide, xylooligosaccharide, oligotose, maltitol, palatinose, and xylitol, and is preferably glucose, fructose, and/or sucrose.
[42] The food product according to any one of [35] to [41], comprising the viable lactic acid bacterium in an amount of 1 × 10² cfu/ml or more.
[43] The food product according to any one of [35] to [42], having a Y₁/X₁ value, calculated from an acidity increase rate X₁ (%) and a bacterial survival rate Y₁ (%) after storage at 25°C for 21 days from production of 0.012 or more.
[44] The food product according to any one of [35] to [43], wherein a Y₂/X₂ value, calculated from an acidity increase rate X₂ (%) and a bacterial survival rate Y₂ (%) after storage at 30°C for 14 days from production is 0.012 or more.
[44] The food product according to any one of [35] to [43], wherein the lactic acid bacterium is of the *Lactobacillus* genus (*Lactocaseibacillus* genus).

### Advantageous Effects of Invention

The present disclosure provides a technique for suppressing increase in acidity and decrease in the number of viable bacteria during storage of a food product containing a viable lactic acid bacterium and containing a small amount of a nonfat milk solid content or protein serving as nutrients for lactic acid bacteria, such as a lactic acid bacteria beverage and a fermented milk-based lactic acid bacteria beverage.

### Description of Embodiments

Now, preferred embodiments for carrying out the present disclosure will be described. The embodiments described below are examples of typical embodiments of the present disclosure, and should not be construed as narrowing the scope of the present disclosure.

### 1. Food Product

A food product of the present disclosure comprises a milk ingredient and a viable lactic acid bacterium, has a nonfat milk solid content of less than 8.0 wt%, a protein content of less than 2.7 wt%, and a pH of 4.6 or less, and comprises an emulsifier and a water activity reducing agent.

In another embodiment, the food product of the present disclosure comprises a milk ingredient and a viable lactic acid bacterium, has a nonfat milk solid content of less than 8.0 wt%, a protein content of less than 2.7 wt%, and a content of fats or oils of less than 10 wt%, comprises an emulsifier having an HLB of 9 or more, and has water activity Aw at 25°C of 0.990 or less.

### [Milk ingredient]

As the milk ingredient, conventionally known milk raw materials may be contained, including, for example, raw milk and processed forms thereof (such as milk, fat-adjusted milk, processed milk, skimmed milk, concentrated milk, concentrated skimmed milk, whole milk powder, prepared milk powder, skimmed milk powder, condensed milk, cream, and butter). One of these milk raw materials may be used, or a plurality of the milk raw materials may be used in combination.

From the viewpoint of achieving a refreshing flavor, the nonfat milk solid content is preferably low, and specifically preferably less than 8.0 wt%. When the nonfat milk solid content, which serves as a nutrient source for lactic acid bacteria, is low, maintaining the number of viable bacteria is particularly difficult, and the effects of the present invention is exhibited remarkably.

The nonfat milk solid content is more preferably 0.1 wt% or more and less than 7 wt%, further preferably 0.5 wt% or more and less than 6.5 wt%, and most preferably 1 wt% or more and less than 4 wt%.

From the viewpoint of achieving good fluidity, the protein content is preferably low, and is specifically preferably less than 2.7 wt%. When the content of protein, which serves as a nutrient source for lactic acid bacteria, is low, maintaining the number of viable bacteria is particularly difficult, and the effect of the present invention is exhibited remarkably.

The protein content is more preferably 0.01 wt% or more and less than 2.5 wt%, further preferably 0.1 wt% or more and less than 2 wt%, and most preferably 0.5 wt% or more and less than 1.5 wt%.

The nonfat milk solid content and protein content can be appropriately set within the aforementioned numerical ranges, depending on desired properties, flavor, and application of the food product.

The nonfat milk solid content and protein content can be calculated as theoretical values using numerical values listed in the Standard Tables of Food Composition in Japan 2020 (Eighth Revised Edition).

Alternatively, the nonfat milk solid content and protein content can be measured according to conventional methods, for example, by methods described in the "Ministerial Ordinance Concerning Standards for Ingredients of Milk and Dairy Products" (Ministerial Ordinance on Milk and Related Products, Ordinance of the Ministry of Health and Welfare No. 52, December 27, 1951), which is a ministerial ordinance based on Japan's Food Sanitation Act, or in the international food standard, CODEX STANDARD FOR FERMENTED MILKS (CODEX STAN 243-2003).

For a desirable acidic taste and excellent flavor, the pH of the food product of the present disclosure is preferably low at the start of storage (for example, at the time or date of completion of all processes for fermented milk production).

A low pH is also preferable because it is less likely to exceed the isoelectric point of milk protein, thus offering excellent stability of the food product. Specifically, the pH is preferably 4.6 or less, more preferably 4.5 or less, further preferably 4.4 or less, particularly preferably 4.2 or less, and most preferably 4.0 or less.

The food product of the present disclosure comprises a viable lactic acid bacterium. Specifically, the food product can be a lactic acid bacteria beverage, a fermented milk-based lactic acid bacteria beverage, or a food product using milk as a main raw material.

A lactic acid bacteria beverage refers to a product that contains a milk ingredient, with a nonfat milk solid content of less than 3 wt% and a protein content of less than 2.7 wt%. This encompasses food products that meet either the definition of Ministerial Ordinance on Milk and Related Products or the definition of the CODEX STANDARD FOR Drinks based on Fermented Milk, and are produced by methods specified in these.

A fermented milk-based lactic acid bacteria beverage refers to a product that contains a milk ingredient, with a nonfat milk solid content of 3 wt% or more and a protein content of less than 2.7 wt%. This encompasses food products that meet either the definition of Ministerial Ordinance on Milk and Related Products or the definition of the CODEX STANDARD FOR Drinks based on Fermented Milk, and are produced by methods specified in these.

It is noted that fermented milk (yogurt) corresponds to neither a lactic acid bacterial beverage nor a fermented milk-based lactic acid bacteria beverage. Fermented milk refers to a product that contains a milk ingredient, with a nonfat milk solid content of 8 wt% or more and a protein content of 2.7 wt% or more. This encompasses food products that meet either the definition of Ministerial Ordinance on Milk and Related Products or the definition of the CODEX STANDARD FOR Drinks based on Fermented Milk, and are produced by methods specified in these. Ministerial Ordinance on Milk and Related Products defines fermented milk as "product obtained by fermenting, with a lactic acid bacterium or yeast, milk or a related product containing a nonfat milk solid content equivalent to or greater than milk into a paste-like or liquid form, or freezing the resultant".

The food product using milk or the like as a main raw material (excluding beverages) refers to a product that contains a milk ingredient, with a nonfat milk solid content of less than 8 wt% and a protein content of less than 2.7 wt%. This encompasses food products that meet either the definition of Ministerial Ordinance on Milk and Related Products or the definition of the CODEX STANDARD FOR Drinks based on Fermented Milk, and are produced by methods specified in these. A food product using milk or the like as a main raw material refers to a food product not meeting the definition of fermented milk by Ministerial Ordinance on Milk and Related Products in that the nonfat milk solid content is less than the standard value.

The food product of the present disclosure may comprise fats or oils. A content of fats or oils is less than 10 wt%, more preferably 7 wt% or less, further preferably 4 wt% or less, and most preferably 0.5 wt% or less. When the content of fats or oils is low, maintaining the number of viable bacteria is particularly difficult, and the effect of the present invention is exhibited remarkably. The lower limit of the content of fats or oils is not especially specified, and no fats or oils may be contained.

Fats or oils are not especially limited, and may be either animal fats or vegetable oils. Examples of animal fats include fish oil, beef tallow, lard, milk fat (butter or anhydrous butter), horse oil, snake oil, egg oil, egg yolk oil, turtle oil, and mink oil. Examples of vegetable oils include soybean oil, corn oil, cottonseed oil, rapeseed oil, sesame oil, perilla oil, rice bran oil, sunflower oil, peanut oil, olive oil, palm oil, rice germ oil, wheat germ oil, brown rice germ oil, adlay oil, garlic oil, macadamia nut oil, avocado oil, evening primrose oil, safflower oil, camellia oil, coconut oil, castor oil, linseed oil, and cocoa butter.

The fats or oils may also be those obtained by subjecting these vegetable oils to fat and oil processing (such as hydrogenation or interesterification). Examples include MCT (medium-chain triglyceride) oil, hydrogenated oils and processed fats such as hydrogenated coconut oil and hydrogenated palm kernel oil, which are obtained by refining, deodorizing, fractionating, hydrogenating, or interesterifying liquid or solid vegetable oils; liquid oils and solid fats obtained by fractionating these fats and oils; and medium-chain fatty acid triglycerides.

MCT (medium-chain triglyceride) oil, milk fat (butter or anhydrous butter), coconut oil, and palm kernel oil are preferred as the fats or oils from the viewpoints of compatibility with fermentation odors and product design.

### [Lactic Acid Bacterium]

The type of lactic acid bacterium is not particularly limited, and may be suitably selected from among the lactic acid bacteria conventionally used as a starter, depending on, for instance, desired properties, flavor, and use of the food product. Examples of such a lactic acid bacterium include lactic acid bacteria of the genus *Lacticaseibacillus,* the genus *Lactobacillus,* the genus *Levilactobacillus,* the genus *Lentilactobacillus,* the genus *Limosilactobacillus,* the genus *Lactiplatibacillus,* the genus *Ligilactobacillus,* the genus *Streptococcus,* and the genus *Bifidobacterium.* These lactic acid bacteria may be used singly or in combinations of two or more thereof.

In 2020, lactic acid bacteria of the *Lactobacillus* genus were subdivided with some of the species renamed (see Zheng et al., "A taxonomic note on the genus Lactobacillus: Description of 23 novel genera, emended description of the genus Lactobacillus Beijerinck 1901, and union of Lactobacillaceae and Leuconostocaceae" Int. J. Syst. Evol. Microbiol. 2020 Apr; 70(4): 2782 - 2858). For instance, the conventional *Lactobacillus* genus was reclassified as the new *Lactocaseibacillus* genus. Hereinafter, the present disclosure will refer to these bacteria using the new classifications established after the reclassification.

Specific examples of the lactic acid bacteria of the genus *Lactocaseibacillus* include *Lactocaseibacillus casei* (*L. casei*), *Lactocaseibacillus rhamnosus* (*L. rhamnosus*), *Lactocaseibacillus paracasei subsp. paracasei* (*L. paracasei subsp. paracasei*), and *Lactocaseibaccilus zeae* (*L. zeae*).

Specific examples of the lactic acid bacterium of the genus *Lactobacillus* include *Lactobacillus bulgaricus* (*Lactobacillus delbrueckii subsp. bulgaricus*), *Lactobacillus acidophilus* (*L. acidophilus), Lactobacillus amylovorus* (*L. amylovorus), Lactobacillus crispatus* (*L. crispatus*), *Lactobacillus delbrueckii subsp. lactis* (*L. delbrueckii subsp. lactis*), *Lactobacillus gallinarum* (*L. gallinarum*), *Lactobacillus gasseri* (*L. gasseri*), *Lactobacillus helveticus* (*L. helveticus), Lactobacillus helveticus subsp. jugurti (L. helveticus subsp. jugurti*), and *Lactobacillus johnsonii* (*L. johnsonii*).

Specific examples of the genus *Levilactobacillus* include *Levilactobacillus brevis* (*L. brevis*).

Specific examples of the genus *Lentilactobacillus* include *Lentilactobacillus buchneri* (*L. buchneri*), *Lentilactobacillus sunkii* (*L. sunkii*), and *Lentilactobacillus kefir* (*L. kefir*).

Specific examples of the genus *Limosilactobacillus* include *Limosilactobacillus fermentum* (*L. fermentum*), *Limosilactobacillus oris* (*L. oris*), and *Limosilactobacillus reuteri* (*L. reuteri*).

Specific examples of the genus *Lactiplantibacillus* include *Lactiplantibacillus paraplantarum* (*L. paraplantarum*), *Lactiplantibacillus pentosus* (*L. pentosus*), and *Lactiplantibacillus plantarum* (*L. plantarum*).

Specific examples of the genus *Ligilactobacillus* include *Ligilactobacillus salivarius* (*L. salivarius*).

Specific examples of the lactic acid bacterium of the genus *Streptococcus* include *Streptococcus salivarius subsp. thermophilus.*

Specific examples of the lactic acid bacterium of the genus *Bifidobacterium* include *Bifidobacterium adolescentis* (*B. adolescentis*), *Bifidobacterium animalis* (*B. animalis*), *Bifidobacterium bifidum* (*B. bifidum*), *Bifidobacterium breve* (*B. breve*), *Bifidobacterium catenulatum* (*B. catenulatum*), *Bifidobacterium globosum* (*B. globusum*), *Bifidobacterium infantis* (*B. infantis*), *Bifidobacterium lactis* (*B. lactis*), *Bifidobacterium longum* (*B. longum*), *Bifidobacterium psudocatenulatum* (*B. pseudocatenulatum*), and *Bifidobacterium suis* (*B. suis*).

The lactic acid bacterium is preferably of the *Lactobacillus* genus or the *Lactocaseibacillus* genus, more preferably *Lactobacillus bulgaricus* or *Lactocaseibacillus paracasei,* and particularly preferably *Lactocaseibacillus paracasei.*

The food product of the present disclosure comprises, at the start of storage (for example, at the time or date of completion of all processes for fermented milk production), the viable lactic acid bacterium in an amount of preferably 1 × 10² cfu/ml or more, more preferably 1 × 10³ cfu/ml or more, further preferably 1 × 10⁴ cfu/ml or more, and particularly preferably 1 × 10⁵ cfu/ml or more.

According to Ministerial Ordinance Concerning Standards for Ingredients of Milk and Dairy Products (Ministerial Ordinance on Milk and Related Products), standards stipulate that a fermented milk-based lactic acid bacteria beverage contains a lactic acid bacterium in an amount of 1 × 10⁷ cfu/ml or more, and that a lactic acid bacteria beverage contains a lactic acid bacterium in an amount of 1 × 10⁶ cfu/ml or more. Accordingly, the food product of the present disclosure may comprise a lactic acid bacterium in an amount of 1 × 10⁶ cfu/ml or more, or 1 × 10⁷ cfu/ml or more.

The number of lactic acid bacteria can be measured by a method specified by Ministerial Ordinance Concerning Standards for Ingredients of Milk and Dairy Products ((Ordinance of the Ministry of Health and Welfare No. 52, 1951) promulgated on December 27, 2019, amended (by Ministry of Health, Labor and Welfare Ordinance No. 87 of 2019)).

### [Emulsifier]

The emulsifier is preferably one or more fatty acid esters selected from the group consisting of a sucrose fatty acid ester, a glycerin fatty acid ester (such as a monoglyceride, an organic acid monoglyceride, or a polyglycerol fatty acid ester), and a stearoyl lactylate (such as sodium stearoyl lactylate or calcium stearoyl lactylate), and is more preferably a sucrose fatty acid ester or a stearoyl lactylate. These emulsifiers may be used singly or in combinations of two or more thereof.

Specific examples of the sucrose fatty acid ester include sucrose laurate (with 12 carbon atoms of fatty acid; the same below), sucrose myristate (with 14 carbon atoms), sucrose palmitate (with 16 carbon atoms), sucrose stearate (with 18 carbon atoms), sucrose oleate (with 18 carbon atoms and 1 double bond), sucrose behenate (with 22 carbon atoms), sucrose erucate (with 22 carbon atoms and 1 double bond), and sucrose mixed fatty acid ester (e.g., mixed fatty acid ester of oleic acid, palmitic acid and stearic acid). Among these, sucrose palmitate is preferred. These sucrose fatty acid esters may be used singly or in combinations of two or more thereof.

A monoester content of the sucrose fatty acid ester is preferably 60 wt% or higher, more preferably 70 wt% or higher, and still more preferably 80 wt% or higher.

The content ratios of triester, monoester, and diester in the sucrose fatty acid ester can be measured by METHOD OF ASSAY described in Residue Monograph prepared by the meeting of the Joint FAO/WHO Expert Committee on Food Additives (JECFA), 84th meeting 2017 "Sucrose Esters of Fatty Acids".

Specifically, a precisely weighed sample is dissolved in a prescribed amount of 100% tetrahydrofuran (HPLC grade), an insoluble matter is then removed therefrom with a 0.5 µm membrane filter, and the resulting solution is used as a sample to conduct high performance liquid chromatography under the conditions described below. Peak areas for monoester, diester, and triester are calculated individually, and a ratio of each to a total peak area of all peaks detected over 50-minute measurement is then calculated.

The peak area corresponds to the area from the start point (rising position) to the end point (falling position) of each peak. When two or more peaks are adjacent and their start or end points are unclear, the area should be calculated by setting a point where the data between the peaks is at its minimum as the start and end points.

### <Measurement Conditions>

Apparatus: Chromaster (manufactured by Hitachi, Ltd.)
Detector: Refractive Index Detector-5450 (manufactured by Hitachi, Ltd.)
Column: TSK gel G2500HXL (manufactured by Tosoh Corporation)
Column temperature: 40°C
Eluent: tetrahydrofuran (100%) 0.8 ml/min
Injection amount: 10 µl

The sucrose fatty acid ester can also be synthesized according to known procedures (for example, by transesterification reaction between sucrose and higher alcohol esters of fatty acids), and various brand (grade) products with different HLBs are available as, for example, "Ryoto (registered trademark) Sugar Ester" manufactured by Mitsubishi Chemical Corporation.

The sucrose fatty acid ester preferably has a high HLB (Hydrophilic-Lipophilic Balance) because this indicates appropriate hydrophilicity and good dispersibility when blended into the raw material composition. Specifically, the HLB is preferably 9 or more, more preferably 12 or more, further preferably 13 or more, and most preferably 15 or more.

The sucrose fatty acid ester is particularly preferably a sucrose palmitate having an HLB of 9 or more.

The HLB can be determined according to known procedures. Methods for calculating HLB include the Atlas method, Griffin method, Davies method, and Kawakami method, and the like, and there is also a determination method based on a retention time in high performance liquid chromatography. For example, in the case (i) where the composition of fatty acid ester compound as a mixture synthesized is known, the HLB of each fatty acid ester compound may be calculated by the Griffin method and the weighted average of the HLBs may be regarded as the HLB of the fatty acid ester compound. In the case (ii) where the composition of fatty acid ester compound is unknown, the HLB of the fatty acid ester compound may be determined by comparing the retention time in high performance liquid chromatography (HPLC) with a sample of fatty acid ester compound with known HLB.

Note that the HLB values of "Ryoto (registered trademark) Sugar Ester" and "Ryoto (registered trademark) Polyglyester" are each indicated in the catalogs (see Mitsubishi Chemical Corporation's website, http://www.mfc.co.jp/product/nyuuka/ryoto_syuga/list.html, https://www.mfc.co.jp/product/nyuuka/ryoto_poriguri/list. html) as approximate (as a numerical value with "about"). Each value can be regarded as the HLB of the corresponding emulsifier (fatty acid ester compound). When another product is used as the sucrose fatty acid ester, the HLB can also refer to the catalog value.

Specific examples of the polyglycerol fatty acid ester include polyglycerol caprylate (with 8 carbon atoms of fatty acid; the same below), polyglycerol laurate (12 carbon atoms), polyglycerol myristate (14 carbon atoms), polyglycerol palmitate (16 carbon atoms), polyglycerol stearate (18 carbon atoms), polyglycerol oleate (18 carbon atoms, 1 double bond), and polyglycerol behenate (22 carbon atoms). Among these, polyglycerol palmitate is preferred.

Although the polyglycerol fatty acid ester may also be synthesized by known procedures (e.g., transesterification between polyglycerol and a higher alcohol ester(s) of fatty acid), various brand (grade) products with different HLBs are available, for example, as "Ryoto (registered trademark) Polyglyester", manufactured by Mitsubishi Chemical Corporation.

The polyglycerol fatty acid ester has an average polymerization degree of preferably 3 or less. The average polymerization degree is more preferably 2.5 or less, and further preferably 2 or less.

Note that the average polymerization degree of polyglycerol fatty acid ester and the constituent fatty acid linked to the polyglycerol fatty acid ester can be measured and identified according to conventional procedures. Examples include a procedure for separating and quantifying a TMSylated and/or acetylated polyglycerol derivative by gas chromatography (GC method). Analysis by the GC method may be performed using a fused silica capillary tube chemically bonded to a low-polarity liquid phase such as methyl silicon, for example, with a temperature increase of 10°C/min from 100°C to 250°C. In addition, the peak(s) of polymerization degree on a gas chromatogram may be identified, for example, by introducing gas chromatography to a double-focusing mass spectrometer, ionizing and measuring a material by chemical ionization or other methods, then determining the molecular weight of the peak on the gas chromatogram from the molecular weight of its parent ion, and further determining the polymerization degree of glycerol from the chemical formula.

Specific examples of the organic acid monoglyceride include diacetyl tartaric acid esters of monoglycerides (DATEM), and succinic acid monoglyceride.

A content of the emulsifier is preferably 0.001 to 5.0 wt%, and more preferably 0.005 to 0.5 wt%.
When the emulsifier is a sucrose fatty acid ester, in particular, the content is preferably 0.001 to 1.0 wt%, more preferably 0.05 to 0.5 wt%, and further preferably 0.08 to 0.20 wt%.
When the emulsifier is a stearoyl lactylate, in particular, the content is preferably 0.001 to 1.0 wt%, more preferably 0.01 to 0.5 wt%, and further preferably 0.05 to 0.15 wt%.

The content of the emulsifier in the food product can be measured according to conventional procedures such as high performance liquid chromatography (HPLC).

### [Water Activity Reducing Agent]

The water activity reducing agent refers to a substance that dissolves in water and lowers the water activity in the food product.

The water activity reducing agent is preferably one or more selected from the group consisting of glucose, xylose, fructose, psicose, sucrose, lactose, erythritol, sorbitol, trehalose, mannitol, isomaltooligosaccharide, xylooligosaccharide, oligotose, maltitol, palatinose, xylitol, sodium chloride, magnesium chloride, calcium lactate, glycerin, ethanol, and isopropanol. The water activity reducing agent is preferably glucose, fructose, sucrose, erythritol, or mannitol, and more preferably glucose, fructose and/or sucrose due to their good flavor.

From the viewpoint of assimilability of the lactic acid bacteria, the water activity reducing agent preferably contains at least glucose as a first water activity reducing agent, and one or more selected from the group consisting of fructose, sucrose, erythritol, sorbitol, mannitol, isomaltooligosaccharide, xylooligosaccharide, oligotose, maltitol, palatinose, and xylitol as a second water activity reducing agent.

A content of the water activity reducing agent is preferably 0.1 to 50 wt%, more preferably 1 to 40 wt%, and further preferably 2 to 35 wt%. The content of the water activity reducing agent is preferably 5 wt% or more, more preferably 10.0 wt% or more, and further preferably 20 wt% or more.

The content of the water activity reducing agent, for example, a sugar like glucose or fructose, in the food product can be measured according to conventional procedures, such as high performance liquid chromatography (HPLC).

The food product of the present disclosure, containing the water activity reducing agent in the aforementioned content, can have water activity Aw at 25°C of 0.990 or less. The water activity at 25°C is more preferably 0.985 or less, further preferably 0.983 or less, most preferably 0.981 or less, and further preferably 0.979 or less. The water activity Aw can be measured with a water activity meter. In the present invention, the "water activity at 25°C" shall include measurement values taken at 24.5°C to 25.4°C.

The food product of the present disclosure comprises the emulsifier and the water activity reducing agent, and hence can suppress the increase in acidity and the decrease in the number of viable lactic acid bacteria even after a certain period of storage, even if the nonfat milk solid and protein contents, and the content of fats or oils, which serve as nutrients for lactic acid bacteria, are low. The combined use of the water activity reducing agent and the emulsifier puts the lactic acid bacterium into a dormant-like state, which probably allows the maintenance of the number of viable bacteria. This makes it possible to suppress the increase in acidity and the decrease in the number of viable lactic acid bacteria even in distribution at room temperature, particularly in regions where refrigerated distribution is underdeveloped, or in situations where the refrigerated distribution has been developed but is difficult to choose due to energy costs or environmental impact. The suppression of the increase in acidity and the decrease in the number of viable lactic acid bacteria during a certain period of storage may be due to metabolic inhibition of the lactic acid bacteria by the emulsifier and the water activity reducing agent.

The suppression of the increase in acidity may be checked, for example, by storing a food product under conditions at a given temperature and for a given period of time, and determining how much the acidity has increased based on acidities at the start (date) and the end (date) of the storage. For example, a food product of the present disclosure and a food product as a control are each stored at a given temperature (for example, 4 to 10°C, 11 to 20°C, or 21 to 30°C) for a given period (for example, 14 days), and the acidity is measured before and after the storage. When the degree of the increase in the food product of the present disclosure is smaller than that in the control product, it can be determined that the effect of suppressing the increase in acidity during storage has been elicited.

Besides, the decrease in the number of viable lactic acid bacteria can be checked, for example, by storing a food product under conditions at a given temperature and for a given period of time, and determining how much the number of bacteria has decreased based on the numbers at the start (date) and the end (date) of storage. For example, a food product of the present disclosure and a food product as a control are each stored at a given temperature (for example, 4 to 10°C, 11 to 20°C, or 21 to 30°C) for a given period (for example, 14 days), and the number of bacteria is measured before and after the storage. When the degree of the decrease in the food product of the present disclosure is smaller than that in the control product, it can be determined that the effect of suppressing the decrease in survival rate of lactic acid bacteria during the storage has been elicited.

The acidity of fermented milk can be measured by a method specified by Ministerial Ordinance Concerning Standards for Ingredients of Milk and Dairy Products ((Ordinance of the Ministry of Health and Welfare No. 52, 1951) promulgated on December 27, 2019, amended (by Ministry of Health, Labor and Welfare Ordinance No. 87 of 2019)).

The survival rate (%) of lactic acid bacteria can be determined by dividing the number of viable bacteria after a certain period of storage by the number of viable bacteria at the start of the storage (for example, at the time or date of completion of all processes for producing fermented milk) to obtain the resultant as a percentage.

The food product of the present disclosure preferably has a high Y/X value, calculated from an acidity increase rate X (%) and a bacterial survival rate Y (%) after storage at 25 to 30°C for 14 to 21 days from the production. Specifically, a Y₁/X₁ value, calculated from an acidity increase rate X₁ (%) and a bacterial survival rate Y₁ (%) after storage at 25°C for 21 days from the production is preferably 0.012 or more, more preferably 0.015 or more, further preferably 0.03 or more, and most preferably 0.04 or more. Besides, a Y₁/X₁ value, calculated from an acidity increase rate X₁ (%) and a bacterial survival rate Y₁ (%) after storage at 30°C for 14 days from the production is preferably 0.012 or more, more preferably 0.015 or more, further preferably 0.03 or more, and most preferably 0.04 or more.

### [Additional Ingredients]

Examples of additional ingredient(s) that can be optionally contained in the food product of the present disclosure include food additives such as colorants, thickeners/stabilizers/gelling agents (e.g., gelatin, agar, pectin, carboxymethyl cellulose (CMC)), and flavoring agents.

### 2. Production Method

A method for producing a food product of the present disclosure is a method for producing a food product that comprises a milk ingredient and a viable lactic acid bacterium, and has a nonfat milk solid content of less than 8.0 wt%, a protein content of less than 2.7 wt%, and a pH of 4.6 or less, and the method comprises a step of fermenting the milk ingredient, and a step of adding a water activity reducing agent and an emulsifier to the milk ingredient. The step of fermenting the milk ingredient and the step of adding a water activity reducing agent and an emulsifier to the milk ingredient can be performed in either order. Besides, the water activity reducing agent and the emulsifier may be added simultaneously or separately to the milk ingredient. The addition of the water activity reducing agent and the emulsifier may be conducted all at once or dividedly in plural times. The method for producing a food product of the present disclosure preferably includes any one of the following steps (1) to (3):
(1) a step of adding the water activity reducing agent to the milk ingredient before fermentation by the lactic acid bacterium, and a step of adding the emulsifier to the milk ingredient after fermentation by the lactic acid bacterium;
(2) a step of adding the water activity reducing agent to the milk ingredient before fermentation by the lactic acid bacterium, and a step of adding the emulsifier and the water activity reducing agent to the milk ingredient after fermentation by the lactic acid bacterium; and
(3) a step of adding the emulsifier and the water activity reducing agent to the milk ingredient after fermentation by the lactic acid bacterium.

The method for producing a food product of the present disclosure can be carried out through the same steps as those of a usual method for producing a food product containing a lactic acid viable bacterium except for the aforementioned step of adding the moisture activity reducing agent and the emulsifier.

A method for producing a food product containing a lactic acid viable bacterium typically includes a step of preparing a raw material composition (preparation step) and a step of adding a lactic acid bacterium starter to the raw material composition for fermentation (fermentation step).

Here, the step (1) of the method for producing a food product of the present disclosure can be carried out by adding a water activity reducing agent to a raw material composition in the preparation step of the conventional production method, fermenting the resultant, and then adding an emulsifier to the resulting fermented milk. The step (2) of the method for producing a food product of the present disclosure can be carried out by adding a partial amount of the water activity reducing agent to the raw material composition in the preparation step, fermenting the resultant, and then adding an emulsifier and the remaining amount of the water activity reducing agent to the resulting fermented milk. The step (3) of the method for producing a food product of the present disclosure can be carried out by adding, after the fermentation step, an emulsifier and a water activity reducing agent to the resulting fermented milk.

In the preparation step of the conventional production method, the milk ingredient and other ingredients described above are typically mixed to obtain the raw material composition. In the method for producing a food product of the present disclosure, the raw material composition is preferably adjusted at this stage so that the nonfat milk solid and protein contents fall within the numerical ranges described above.

In the fermentation step, fermentation is carried out usually at 30 to 50°C, and preferably 40 to 45°C, usually for 1 to 24 hours, and preferably for 3 to 24 hours. In the method for producing a food product of the present disclosure, the lactic acid bacterium starter, and fermentation temperature and time are preferably adjusted at this stage so that the number of lactic acid bacteria and pH fall within the numerical ranges described above. The raw material composition may also be sterilized according to a conventional method before starting the fermentation.

According to the method for producing a food product of the present disclosure, since the emulsifier and the water activity reducing agent are blended, it is possible to obtain a food product containing viable lactic acid bacteria in which the increase in acidity and the decrease in the number of viable lactic acid bacteria are suppressed even after a certain period of storage.

### 3. Method for Suppressing Increase in Acidity and/or Decrease in Number of Viable Bacteria

A method for suppressing increase in acidity and/or decrease in the number of viable bacteria after production in a food product of the present disclosure is a method for suppressing increase in acidity and/or decrease in the number of viable bacteria after production in a food product that comprises a milk ingredient and a viable lactic acid bacterium, and has a nonfat milk solid content of less than 8.0 wt%, a protein content of less than 2.7 wt%, and a pH of 4.6 or less. The method comprises a step of fermenting the milk ingredient, and a step of adding a water activity reducing agent and an emulsifier to the milk ingredient. The step of fermenting the milk ingredient and the step of adding a water activity reducing agent and an emulsifier to the milk ingredient can be performed in either order. Besides, the water activity reducing agent and the emulsifier may be added simultaneously or separately.

The addition of the water activity reducing agent and the emulsifier may be conducted all at once or dividedly in plural times. The method for producing a food product of the present disclosure preferably includes any one of the steps (1) to (3) described above.

In the method for suppressing increase in acidity and/or decrease in the number of viable bacteria after production in a food product of the present disclosure, the technical matters related to the method for producing a food product of the present disclosure described above can be appropriately applied.

### 4. Use of Emulsifier and Water Activity Reducing Agent

As described above, the food product of the present disclosure comprises the emulsifier and the water activity reducing agent in the amounts within the above-described ranges, and hence the increase in acidity and the decrease in the number of viable lactic acid bacteria can be suppressed even after a certain period of storage. In other words, use of an emulsifier and a water activity reducing agent of the present disclosure is for suppressing increase in acidity and/or decrease in the number of viable bacteria after production in a food product that comprises a milk ingredient and a viable lactic acid bacterium, and has a nonfat milk solid content of less than 8.0 wt%, a protein content of less than 2.7 wt%, and a pH of 4.6 or less.

### Examples

Products and measurement methods used in the following examples are as follows, unless otherwise specified.
Emulsifier: sucrose fatty acid ester "Ryoto Sugar Ester P-1670" (Mitsubishi Chemical Corporation, sucrose palmitate, HLB = 16, monoester content: 80 wt%)
New Yakult (Yakult Honsha Co., Ltd.)
Lactic acid bacteria FD-DVS *L. casei*-01 (Chr. Hansen A/S)
Sugar (granulated sugar, Nissin Seito)
High fructose corn syrup (Fuji Fruct F-100, NIHON SHOKUHIN KAKO CO., LTD.)
Skimmed milk (MORINAGA MILK INDUSTRY CO., LTD.)
Anhydrous dextrose (Showa Sangyo Co., Ltd.)

### (1) Calculation of Nonfat Milk Solid Content and Protein Content

A theoretical value for each composition was calculated using values listed in the Standard Tables of Food Composition in Japan 2020 (8th revised edition).

### (2) Measurement of Number of Lactic Acid Bacteria and Calculation of Survival Rate

The number of lactic acid bacteria was measured with appropriate conditions set by referring to Ministerial Ordinance Concerning Standards for Ingredients of Milk and Dairy Products ((Ordinance of the Ministry of Health and Welfare No. 52, 1951) promulgated on December 27, 2019, amended (by Ministry of Health, Labor and Welfare Ordinance No. 87 of 2019)).

The survival rate (%) was determined by dividing the number of viable bacteria after a certain period of storage by the number of viable bacteria immediately after preparation (day 0 of storage) to obtain the resultant as a percentage.

### (3) Measurement of Acidity

The measurement was carried out according to the method described in Ministerial Ordinance Concerning Standards for Ingredients of Milk and Dairy Products ((Ordinance of the Ministry of Health and Welfare No. 52, 1951) promulgated on December 27, 2019, amended (by Ministry of Health, Labor and Welfare Ordinance No. 87 of 2019)) to calculate the acidity as lactic acidity (%). An acidity increase rate (%) was determined by dividing acidity after a certain period of storage by acidity immediately after preparation (day 0 of storage) to obtain the resultant as a percentage.

### (4) Measurement of Water Activity Aw

Water activity Aw was measured with AquaLab Series 4TDL (manufactured by METER).

### [Example 1]

### (1) Preparation of Mother Starter

15 wt% of skimmed milk powder, 8 wt% of high fructose corn syrup, and 74 wt% of water were mixed and dissolved.

The thus obtained mixed solution was sterilized at 100°C for 1 hour, and then cooled to 37°C.

After adding and mixing 3 wt% of New Yakul to and with the mixed solution, the resultant was fermented at 37°C for 48 hours to obtain a mother starter. The mother starter was frozen in liquid nitrogen and stored frozen.

### (2) Preparation of Lactic Acid Bacteria Beverage

A skimmed milk powder and a water activity reducing agent (high fructose corn syrup) were dissolved in water, and the mother starter, which had been returned to room temperature, was added thereto, and the resultant was fermented at 35°C to obtain a fermented milk base.

To the thus obtained fermented milk base, an emulsifier (sucrose palmitate) and water activity reducing agents (sugar and high fructose corn syrup) were added to be mixed so as to achieve a final composition shown in Table 1. The resultant mixture was filled into a plastic container and cooled overnight to obtain a lactic acid bacteria beverage (Example 1). The lactic acid bacteria beverage thus obtained had a nonfat milk solid content of 3.43 wt%, a protein content of 1.2 wt%, and a pH of 3.66.

### [Comparative Example 1]

A lactic acid bacteria beverage was obtained in the same manner as in Example 1 except that sucrose palmitate was not added in the preparation of the lactic acid bacteria beverage of Example 1. The thus obtained lactic acid bacteria beverage had a pH of 3.65.

**[Table 1]**

| | Comparative Example 1 | Example 1 |
|---|---|---|
| Skimmed Milk Powder | 3.60 wt% | 3.60 wt% |
| Sugar | 3.04 wt% | 3.04 wt% |
| High Fructose Corn Syrup | 17.60 wt% | 17.60 wt% |
| Sucrose Fatty Acid Ester | (none) | 0.114 wt% |

Tables 2 and 3 show the results of measurement of the acidity and calculation of the survival rate of the lactic acid bacteria beverages of Example 1 and Comparative Example 1, after storage at 25°C for 21 days or at 30°C for 14 days.

**[Table 2]**

| | Comparative Example 1 | Example 1 |
|---|---|---|
| Number of Viable Bacteria (cfu/ml) after storage for 0 day | 4.7E+08 | 3.1E+08 |
| Number of Viable Bacteria (cfu/ml) after storage at 25°C for 21 days | 6.50E+06 | 1.50E+07 |
| Survival Rate (after storage for 21 days/after storage for 0 day) | 1.38% | 4.84% |
| Acidity after storage for 0 day | 0.62% | 0.61% |
| Acidity after storage at 25°C for 21 days | 0.80% | 0.74% |
| Acidity Increase Rate (after storage for 21 days/after storage for 0 day) | 129% | 121% |
| Bacterial Survival Rate /Acidity Increase Rate | 0.011 | 0.040 |

**[Table 3]**

| | Comparative Example 1 | Example 1 |
|---|---|---|
| Number of Viable Bacteria (cfu/ml) after storage for 0 day | 4.7E+08 | 3.1E+08 |
| Number of Viable Bacteria (cfu/ml) after storage at 30°C for 14 days | 4.1E+06 | 1.8E+07 |
| Survival Rate (after storage for 14 days/after storage for 0 day) | 0.87% | 5.8% |
| Acidity after storage for 0 day | 0.62% | 0.61% |
| Acidity 30°C after storage for 14 days | 0.80% | 0.77% |
| Acidity Increase Rate (after storage for 14 days/after storage for 0 day) | 129% | 126% |
| Bacterial Survival Rate /Acidity Increase Rate | 0.0067 | 0.046 |

Under storage conditions of 21 days at 25°C and 14 days at 30°C, the lactic acid bacteria beverage of Example 1 showed suppressed the increase in acidity and significantly improved survival rate compared to the lactic acid bacteria beverage of Comparative Example 1. In other words, it has been confirmed that the food product of the present disclosure can suppress the increase in acidity and the decrease in the number of viable bacteria during storage, despite the low nonfat milk solid and protein contents.

### [Example 2]

A test was carried out similarly to Example 1 with the lactic acid bacteria species changed.

### (1) Preparation of Lactic Acid Bacteria Beverage

Skimmed milk powder and a water activity reducing agent (high fructose corn syrup) were dissolved in water, powdered lactic acid bacteria (FD-DVS *L.casei*-01) was added as a starter thereto, and the resultant was fermented at 37°C to obtain a fermented milk base.

To the thus obtained fermented milk base, an emulsifier (sucrose palmitate), water activity reducing agents (sugar, high fructose corn syrup), and a stabilizer (pectin) were added to be mixed so as to achieve a final composition shown in Table 4. The resultant mixture was filled into a plastic container and cooled overnight to obtain a lactic acid bacteria beverage (Example 2). The lactic acid bacteria beverage thus obtained had a nonfat milk solid content of 3.43 wt%, a protein content of 1.2 wt%, and a pH of 3.81.

### [Comparative Example 2]

A lactic acid bacteria beverage was obtained in the same manner as in Example 2 except that sucrose palmitate was not added in the preparation of the lactic acid bacteria beverage of Example 2. The thus obtained lactic acid bacteria beverage had a pH of 3.79.

**[Table 4]**

| | Comparative Example 2 | Example 2 |
|---|---|---|
| Skimmed Milk Powder | 3.60 wt% | 3.60 wt% |
| Sugar | 3.04 wt% | 3.04 wt% |
| High Fructose Corn Syrup | 17.60 wt% | 17.60 wt% |
| Pectin | 0.25 wt% | 0.25 wt% |
| Sucrose Palmitate | (none) | 0.15 wt% |

Tables 5 to 7 show the results of measurement of the acidity of these lactic acid bacteria beverages, after storage at 10°C, 25°C, or 30°C for 20 days.

**[Table 5]**

| | Comparative Example 2 | Example 2 |
|---|---|---|
| Acidity after storage for 0 day | 0.49% | 0.48% |
| Acidity after storage at 10° for 20 days | 0.67% | 0.51% |
| Acidity Increase Rate (after storage for 20 days/after storage for 0 day) | 137% | 106% |

**[Table 6]**

| | Comparative Example 2 | Example 2 |
|---|---|---|
| Acidity after storage for 0 day | 0.49% | 0.48% |
| Acidity 25°C after storage for 20 days | 0.99% | 0.79% |
| Acidity Increase Rate (after storage for 20 days/after storage for 0 day) | 202% | 165% |

**[Table 7]**

| | Comparative Example 2 | Example 2 |
|---|---|---|
| Acidity after storage for 0 day | 0.49% | 0.48% |
| Acidity 30°C after storage for 20 days | 1.11% | 0.86% |
| Acidity Increase Rate (after storage for 20 days/after storage for 0 day) | 227% | 179% |

Under storage conditions of 20 days at 10°C, 25°C, and 30°C, the lactic acid bacteria beverage of Example 2 showed suppressed the increase in acidity compared to the lactic acid bacteria beverage of Comparative Example 2. In other words, it has been confirmed that the food product of the present disclosure can suppress the increase in acidity during storage, despite the low nonfat milk solid and protein contents.

### [Example 3]

A lactic acid bacteria beverage was prepared in the same manner as in Example 2 except that the high fructose corn syrup added as the water activity reducing agent in the preparation of the fermented milk base was changed to glucose, and that the sugar and the high fructose corn syrup added to the prepared fermented milk base as the water activity reducing agents were changed to sugar alone.

### (1) Preparation of Lactic Acid Bacteria Beverage

Skimmed milk powder and a water activity reducing agent (glucose) were dissolved in water, powdered lactic acid bacteria (FD-DVS *L.casei-01)* were added as a starter thereto, and the resultant was fermented at 37°C to obtain a fermented milk base.

To the thus obtained fermented milk base, an emulsifier (sucrose palmitate), a water activity reducing agent (sugar), and a stabilizer (pectin) were added to be mixed so as to achieve a final composition shown in Table 8. The resultant mixture was filled into a plastic container and cooled overnight to obtain a lactic acid bacteria beverage (Example 3). The lactic acid bacteria beverage thus obtained had a nonfat milk solid content of 3.43 wt%, a protein content of 1.2 wt%, and a pH of 3.81.

### [Comparative Example 3]

A lactic acid bacteria beverage was obtained in the same manner as in Example 3 except that sucrose palmitate was not added in the preparation of the lactic acid bacteria beverage of Example 3. The thus obtained lactic acid bacteria beverage had a pH of 3.81.

**[Table 8]**

| | Comparative Example 3 | Example 3 |
|---|---|---|
| Skimmed Milk Powder | 3.6 wt% | 3.6 wt% |
| Sugar | 13.68 wt% | 13.68 wt% |
| Glucose | 1.70 wt% | 1.70 wt% |
| Pectin | 0.25 wt% | 0.25 wt% |
| Sucrose Palmitate | (none) | 0.15 wt% |

Table 9 shows the results of measurement of the acidity of these lactic acid bacteria beverages, obtained after storage at 10°C for 20 days.

**[Table 9]**

| | Comparative Example 3 | Example 3 |
|---|---|---|
| Acidity after storage for 0 day | 0.48% | 0.49% |
| Acidity 10°C after storage for 21 days | 0.66% | 0.58% |
| Acidity Increase Rate (after storage for 21 days/after storage for 0 day) | 138% | 118% |

The lactic acid bacteria beverage of Example 3 showed suppressed increase in acidity compared to the lactic acid bacteria beverage of Comparative Example 3.

Table 10 shows the results of measurement of the acidity and calculation of the survival rate of these lactic acid bacteria beverages, obtained after storage at 25°C for 20 days.

**[Table 10]**

| | Comparative Example 3 | Example 3 |
|---|---|---|
| Number of Viable Bacteria (cfu/ml) after storage for 0 day | 1.1E+09 | 7.6E+08 |
| Number of Viable Bacteria (cfu/ml) after storage at 25°C for 20 days | 2.2E+07 | 2.6E+07 |
| Survival Rate (after storage for 20 days/after storage for 0 day) | 2% | 3.4% |
| Acidity after storage for 0 day | 0.48% | 0.49% |
| Acidity 25°C after storage for 20 days | 0.89% | 0.85% |
| Acidity Increase Rate (after storage for 20 days/after storage for 0 day) | 185% | 173% |
| Bacterial Survival Rate /Acidity Increase Rate | 0.011 | 0.020 |

The lactic acid bacteria beverage of Example 3 showed suppressed increase in acidity, and a remarkably improved survival rate compared to the lactic acid bacteria beverage of Comparative Example 3. In other words, it has been confirmed that the food product of the present disclosure can suppress the increase in acidity and the decrease in the number of viable bacteria during storage, despite the low nonfat milk solid and protein contents.

### [Test Example 1: Study of Type and Concentration of Emulsifier]

### 1. Study of Sucrose Fatty Acid Ester

A test was carried out similarly to Example 2 with the type of the emulsifier (sucrose fatty acid ester) changed.

Skimmed milk powder and a water activity reducing agent (high fructose corn syrup) were dissolved in water, powdered lactic acid bacteria (FD-DVS *L.casei*-01) were added as a starter thereto, and the resultant was fermented at 37°C to obtain a fermented milk base.

To the thus obtained fermented milk base, any one of emulsifiers, water activity reducing agents (sugar, high fructose corn syrup), and a stabilizer (pectin) were added to be mixed so as to achieve a final composition shown in Table 11. The resultant mixture was filled into a plastic container and cooled overnight to obtain a lactic acid bacteria beverage. The lactic acid bacteria beverage thus obtained had a nonfat milk solid content of 3.43 wt%, and a protein content of 1.2 wt%. Table 11 shows the pHs of the lactic acid bacteria beverages thus obtained.

The followings were used as the emulsifier:
"Ryoto Sugar Ester S-1170" (Mitsubishi Chemical Corporation, sucrose stearate, HLB = 11);
"Ryoto Sugar Ester S-1670" (Mitsubishi Chemical Corporation, sucrose stearate, HLB = 16);
"Ryoto Sugar Ester L-1695" (Mitsubishi Chemical Corporation, sucrose laurate, HLB = 16);
"Ryoto Sugar Ester M-1695" (Mitsubishi Chemical Corporation, sucrose myristate, HLB = 16);
"Ryoto Sugar Ester O-1570" (Mitsubishi Chemical Corporation, sucrose oleate, HLB = 15);
"Ryoto Sugar Ester P-1670" (Mitsubishi Chemical Corporation, sucrose palmitate, HLB = 16).

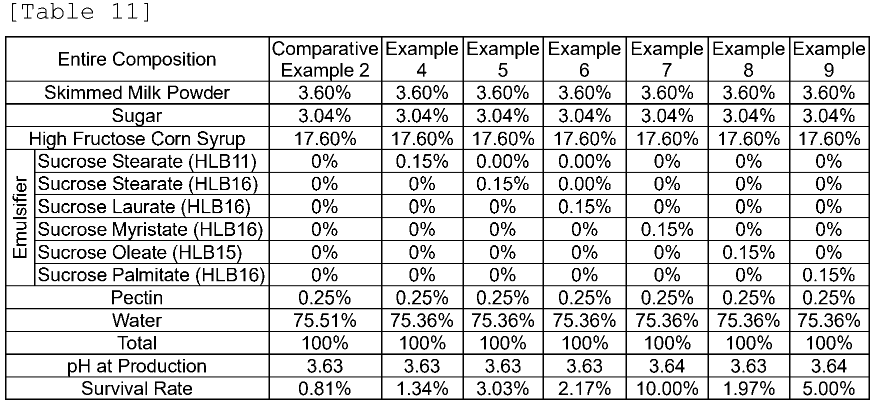

Table 11 shows the results of the survival rate (i.e., the rate of the number of viable bacteria after 35 days storage to the number of viable bacteria after 0 days storage) calculated after storing the lactic acid bacteria beverage at 25°C for 35 days. Table 11 also shows the results of Comparative Example 2 (no emulsifier added).

The survival rate was improved in Examples 4 to 9 compared to Comparative Example 2, and the survival rate was remarkably improved particularly in Examples 7 and 9 in which sucrose myristate (HLB = 16) and sucrose palmitate (HLB = 16) were respectively used.

Besides, tests were carried out similarly to Example 9 with the blending amount of sucrose palmitate (HLB = 16) changed. Table 12 shows the compositions and pHs of the lactic acid bacteria beverages thus obtained.

**[Table 12]**

| Entire Composition | Comparative Example 2 | Example 10 | Example 11 | Example 12 | Example 13 | Example 14 | Example 15 | Example 16 |
|---|---|---|---|---|---|---|---|---|
| Skimmed Milk Powder | 3.60% | 3.60% | 3.60% | 3.60% | 3.60% | 3.60% | 3.60% | 3.60% |
| Sugar | 3.04% | 3.04% | 3.04% | 3.04% | 3.04% | 3.04% | 3.04% | 3.04% |
| High Fructose Corn Syrup | 17.60% | 17.60% | 17.60% | 17.60% | 17.60% | 17.60% | 17.60% | 17.60% |
| Sucrose Palmitate (P-1670) | 0% | 0.08% | 0.10% | 0.12% | 0.14% | 0.16% | 0.18% | 0.20% |
| Pectin | 0.25% | 0.25% | 0.25% | 0.25% | 0.25% | 0.25% | 0.25% | 0.25% |
| Water | 75.51% | 75.43% | 75.41% | 75.39% | 75.37% | 75.35% | 75.33% | 75.31% |
| Total | 100% | 100% | 100% | 100% | 100% | 100% | 100% | 100% |
| pH at Production | 3.61 | 3.6 | 3.6 | 3.6 | 3.6 | 3.6 | 3.6 | 3.6 |
| Survival Rate | 1.51% | 9.33% | 10.83% | 13.64% | 25.00% | 36.84% | 34.21% | 39.47% |
| Increase in Acidity | 134% | 123% | 122% | 123% | 121% | 119% | 119% | 119% |

Table 12 shows the results of the acidity increase rate (i.e., the rate of the acidity after 29 days storage to the acidity after 0 days storage) and the survival rate (i.e., the rate of the number of viable bacteria after 29 days storage to the number of viable bacteria after 0 days storage) calculated after storing the lactic acid bacteria beverage at 25°C for 29 days. Table 12 also shows the results of Comparative Example 2 (no emulsifier added).

All the blending amounts of Examples 10 to 16 showed the effect of suppressing increase in acidity and the effect of improving survival rate.

### 2. Study of Emulsifier except Sucrose Fatty Acid Ester

Tests were carried out similarly to Example 2 with the following emulsifiers, which are not sucrose fatty acid esters, used. Table 13 shows compositions and pHs of lactic acid bacteria beverages thus obtained.
"DATEM517K" (Danisco, Inc., diacetyl tartrate monoglyceride, expressed as "DATEM" in the table);
"GRINSTED SSL FP55" (Danisco, Inc., sodium stearoyl lactylate, expressed as "SSL" in the table);
"Ryoto Polyglyester M-7D" (Mitsubishi Chemical Corporation, polyglycerol myristate, expressed as "M-7D" in the table);
"Poem B-30" (RIKEN VITAMIN CO., LTD., succinic acid monoglyceride, expressed as "succinic acid MG" in the table); and
"TRP-97RF" (RIKEN VITAMIN CO., LTD., triglycerol palmitate, expressed as "TP" in the table).

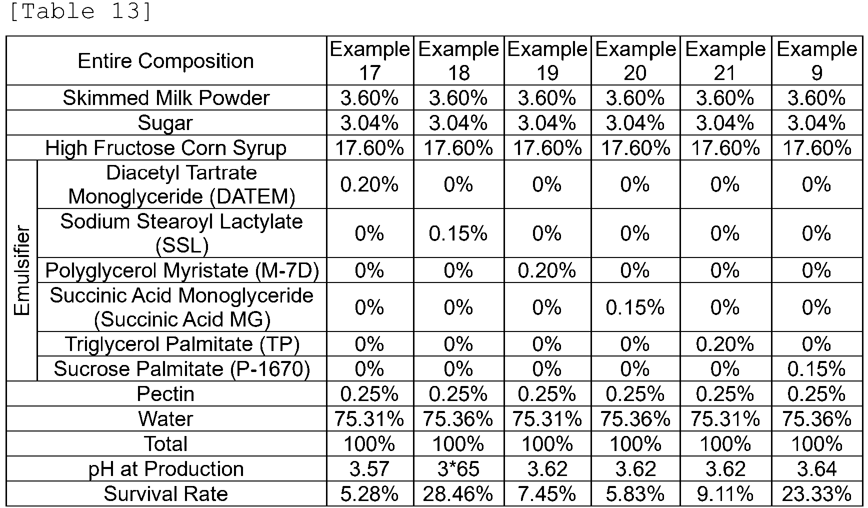

Table 13 shows the results of the survival rate (i.e., the rate of the number of viable bacteria after 28 days storage to the number of viable bacteria after 0 days storage) calculated after storing the lactic acid bacteria beverage at 25°C for 28 days. Table 13 also shows the result of Example 9.

Examples 17 to 21 all showed good survival rates (see Table 12, data of 29 days storage of Comparative Example 2), and in particular, Example 18, in which sodium stearoyl lactylate (SSL) was used, exhibited a remarkably improved survival rate comparable to Example 9, in which sucrose palmitate (HLB = 16) was used.

Furthermore, tests were carried out similarly with "DP-95RF" (RIKEN VITAMIN CO., LTD., diglycerol palmitate, expressed as "DP" in the table) used as the emulsifier, and with the blending amounts of TP and SSL changed from those used above. Table 14 shows compositions and pHs of lactic acid bacteria beverages thus obtained.

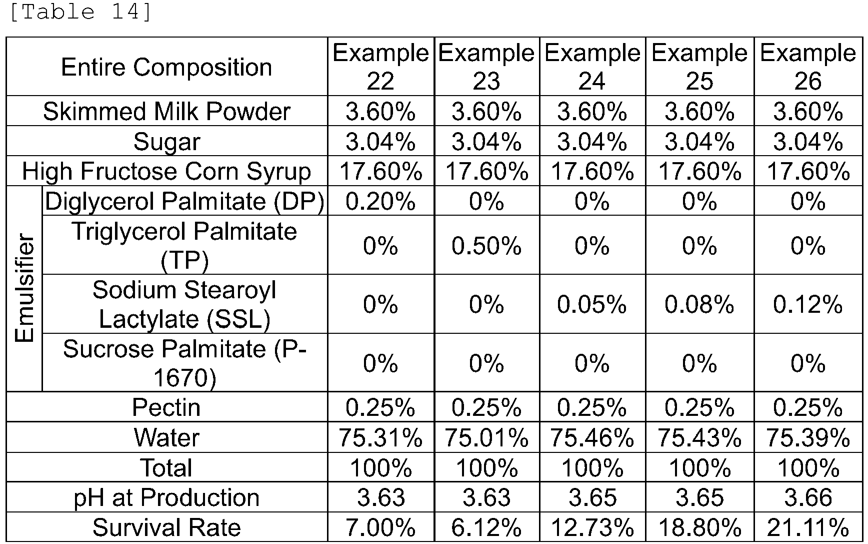

Table 14 shows the results of the survival rate (i.e., the rate of the number of viable bacteria after 28 days storage to the number of viable bacteria after 0 days storage) calculated after storing the lactic acid bacteria beverage at 25°C for 28 days.

Examples 22 to 26 showed good survival rates (see Table 12, data of 29 days storage of Comparative Example 2). The survival rate was remarkably improved by all the blending amounts of Examples 24 to 26, in which sodium stearoyl lactylate (SSL) was used.

### [Test Example 2: Study of Type and Concentration of Water Activity Reducing Agent]

Tests were carried out similarly to Example 2 with the type of the water activity reducing agent changed.

Skimmed milk powder and a water activity reducing agent (high fructose corn syrup) were dissolved in water, powdered lactic acid bacteria (FD-DVS *L.casei*-01) were added as a starter thereto, and the resultant was fermented at 37°C to obtain a fermented milk base. To the thus obtained fermented milk base, an emulsifier (sucrose palmitate), any of various water activity reducing agents, and a stabilizer (pectin) were added to be mixed so as to achieve a final composition shown in Table 15. Also a sample containing no water activity reducing agent was prepared. The resultant mixture was filled into a plastic container and cooled overnight to obtain a lactic acid bacteria beverage. The lactic acid bacteria beverage thus obtained had a nonfat milk solid content of 3.43 wt%, and a protein content of 1.2 wt%. Table 15 shows the pHs of the lactic acid bacteria beverages thus obtained, and Table 16 shows water activity values thereof.

The followings were used as the water activity reducing agent:
xylitol (Xylitol, Bussan Food Science Co., Ltd.);
fructose (Crystalline Fructose M, ADM Japan Ltd.);
xylooligosaccharide (Xylooligosaccharide 95PN, Bussan Food Science Co., Ltd.); and
maltitol (Marbit, Bussan Food Science Co., Ltd.).

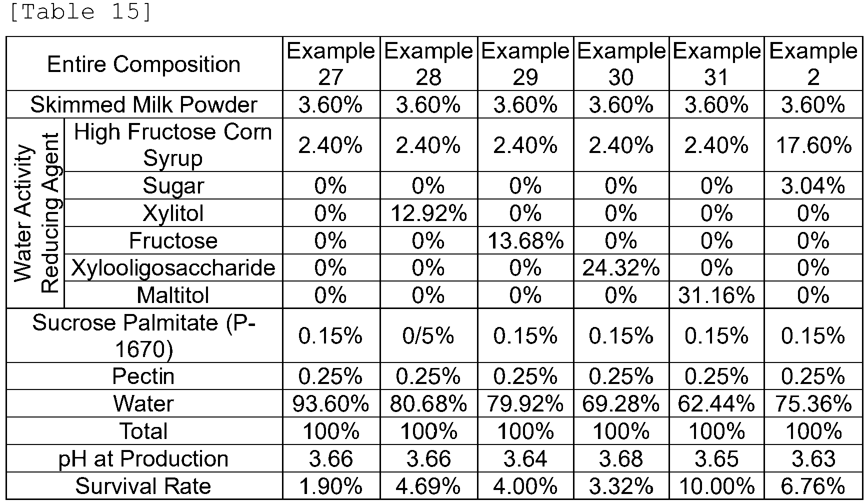

**[Table 16]**

| | Temperature (°C) | Aw |
|---|---|---|
| Example 27 | 24.83 | 0.9959 |
| Example 28 | 24.94 | 0.9789 |
| Example 29 | 24.77 | 0.9789 |
| Example 30 | 24.8 | 0.9765 |
| Example 31 | 24.82 | 0.9787 |
| Example 2 | 24.85 | 0.9803 |

Table 15 shows the results of the survival rate (i.e., the rate of the number of viable bacteria after 34 days storage to the number of viable bacteria after 0 days storage) calculated after storing the lactic acid bacteria beverage at 25°C for 34 days. Table 15 also shows the result of Example 2 (sugar, high fructose corn syrup).

The improvement of the survival rate was confirmed in Examples 27 to 31. The improvement of the survival rate was more remarkable in Examples 28 to 31 and Example 2 in which Aw was 0.990 or less.

Tests were carried out similarly with any of the following water activity reducing agents used. Table 17 shows compositions and pHs of lactic acid bacteria beverages thus obtained, and Table 18 shows water activity values thereof.
Sucrose (granulated sugar, Nissin Seito);
palatinose (PUREPARA, Mitsui DM Sugar Co., Ltd.);
isomaltooligosaccharide (Oligotime, Showa Sangyo Co., Ltd.); and
oligotose (Oligotose, Mitsubishi Chemical Corporation).

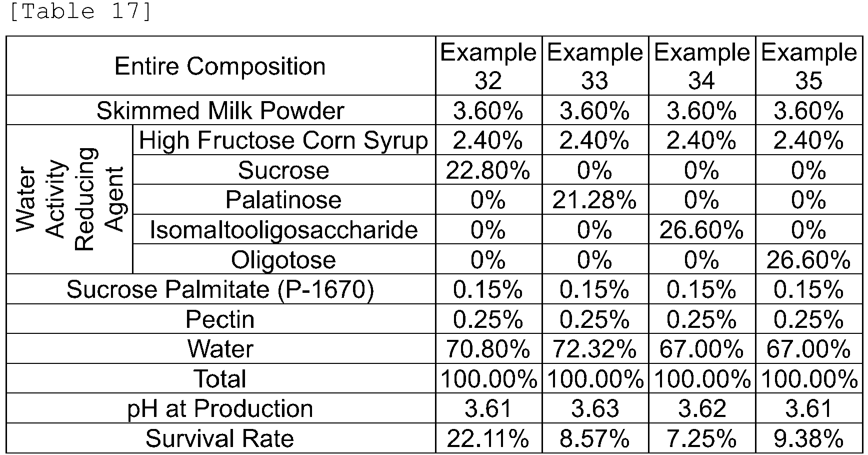

**[Table 18]**

| | Temperature (°C) | Aw |
|---|---|---|
| Example 32 | 24.82 | 0.9818 |
| Example 33 | 24.81 | 0.9804 |
| Example 34 | 24.79 | 0.9825 |
| Example 35 | 24.92 | 0.9781 |

Table 17 shows the results of the survival rate (i.e., the rate of the number of viable bacteria after 28 days storage to the number of viable bacteria after 0 days storage) calculated after storing the lactic acid bacteria beverage at 25°C for 28 days.

The improvement of the survival rate was confirmed in Examples 32 to 35, and the effect was remarkable particularly in using sucrose (Example 32).

Furthermore, lactic acid bacteria beverages were prepared in the same manner as in Example 3 except that the sugar used as the water activity reducing agent added to the prepared fermented milk base was changed to glucose.

Skimmed milk powder and a water activity reducing agent (glucose) were dissolved in water, powdered lactic acid bacteria (FD-DVS *L.casei-01)* were added as a starter thereto, and the resultant was fermented at 37°C to obtain a fermented milk base. To the thus obtained fermented milk base, an emulsifier (sucrose palmitate), a water activity reducing agent (glucose), and a stabilizer (pectin) were added to be mixed so as to achieve a final composition shown in Table 19. The resultant mixture was filled into a plastic container and cooled overnight to obtain a lactic acid bacteria beverage. The lactic acid bacteria beverage thus obtained had a nonfat milk solid content of 3.43 wt%, and a protein content of 1.2 wt%. Table 19 shows the pHs of the lactic acid bacteria beverages thus obtained, and Table 20 shows water activity values thereof.

**[Table 19]**

| Entire Composition | Example 36 | Example 37 | Example 38 | Example 39 |
|---|---|---|---|---|
| Skimmed Milk Powder | 3.60% | 3.60% | 3.60% | 3.60% |
| Glucose | 1.69% | 8.08% | 13.08% | 18.08% |
| Sucrose Palmitate (P-1670) | 0.15% | 0.15% | 0.15% | 0.15% |
| Pectin | 0.25% | 0.25% | 0.25% | 0.25% |
| Water | 94.31% | 87.92% | 82.92% | 77.92% |
| Total | 100% | 100% | 100% | 100% |
| pH at Production | 3.67 | 3.65 | 3.63 | 3.61 |
| Survival Rate | 1.34% | 2.04% | 4.86% | 18.82% |

**[Table 20]**

| | Temperature (°C) | Aw |
|---|---|---|
| Example 36 | 24.78 | 0.9943 |
| Example 37 | 24.85 | 0.9858 |
| Example 38 | 24.85 | 0.9806 |
| Example 39 | 24.83 | 0.9733 |

Table 19 shows the results of the survival rate (i.e., the rate of the number of viable bacteria after 35 days storage to the number of viable bacteria after 0 days storage) calculated after storing the lactic acid bacteria beverage at 25°C for 35 days.

The improvement of the survival rate was confirmed in Examples 36 to 39. The improvement of the survival rate was remarkable particularly in Examples 37 to 39 in which the water activity reducing agent was added in an amount of 2% or more.

### [Test Example 3: Verification of Effect of Combined Use of Emulsifier and Water Activity Reducing Agent]

A lactic acid bacteria beverage was obtained using the same method and composition as in Example 2 (Example 40). Besides, a lactic acid bacteria beverage was obtained using the same water activity reducing agent (high fructose corn syrup) for the preparation of the fermented milk base as in Example 2, but without adding the water activity reducing agents (sugar, high fructose corn syrup) to the fermented milk base (Example 41).

Skimmed milk powder and a water activity reducing agent (high fructose corn syrup) were dissolved in water, powdered lactic acid bacteria (FD-DVS *L.casei*-01) were added as a starter thereto, and the resultant was fermented at 37°C to obtain a fermented milk base.

To the thus obtained fermented milk base, an emulsifier, water activity reducing agents (sugar and high fructose corn syrup), and a stabilizer (pectin) were added to be mixed so as to achieve a final composition shown in Table 21. The resultant mixture was filled into a plastic container and cooled overnight to obtain a lactic acid bacteria beverage. The lactic acid bacteria beverage thus obtained had a nonfat milk solid content of 3.43 wt%, and a protein content of 1.2 wt%. Table 21 shows the pHs of the lactic acid bacteria beverages thus obtained.

### [Comparative Example 3]

A lactic acid bacteria beverage was obtained in the same manner as in Example 40 except that sucrose palmitate was not added in the preparation of the lactic acid bacteria beverage. Table 21 shows the pH of the lactic acid bacteria beverage thus obtained.

### [Comparative Example 4]

A lactic acid bacteria beverage was obtained in the same manner as in Example 41 except that sucrose palmitate was not added in the preparation of the lactic acid bacteria beverage. Table 21 shows the pH of the lactic acid bacteria beverage thus obtained, and Table 22 shows the water activity value thereof.

Table 21 shows the results of the acidity increase rate (i.e., the rate of the acidity after 29 days storage to the acidity after 0 days storage) calculated after storing the lactic acid bacteria beverage at 25°C for 29 days.

**[Table 21]**

| Entire Composition | Comparative Example 3 | Example 40 | Comparative Example 4 | Example 41 |
|---|---|---|---|---|
| Skimmed Milk Powder | 3.60% | 3.60% | 3.60% | 3.60% |
| Sucrose | 3.04% | 3.04% | 0% | 0% |
| High Fructose Corn Syrup | 17.60% | 17.60% | 2.40% | 2.40% |
| Sucrose Fatty Acid Ester (P-1670) | 0% | 0.15% | 0% | 0.15% |
| Pectin | 0.25% | 0.25% | 0.25% | 0.25% |
| Water | 75.51% | 75.36% | 93.75% | 93.60% |
| Tota | 100% | 100% | 100% | 100% |
| pH at Production | 3.63 | 3.64 | 3.68 | 3.68 |
| Acidity Increase Rate | 170.1% | 146.6% | 167.0% | 166.4% |

**[Table 22]**

| | Temperature (°C) | Aw |
|---|---|---|
| Comparative Example 3 | 24.93 | 0.9787 |
| Example 40 | 24.90 | 0.9793 |
| Comparative Example 4 | 24.92 | 0.9957 |
| Example 41 | 24.96 | 0.9919 |

In Comparative Example 4 in which neither water activity reducing agent nor emulsifier was used in the preparation of the lactic acid bacteria beverage, the acidity increase rate was 167.0%.

In Example 41 in which the emulsifier was used in the preparation of the lactic acid bacteria beverage, the acidity increase rate was 166.4%.

In Comparative Example 3 in which the water activity reducing agent was used in the preparation of the lactic acid bacteria beverage, the acidity increase rate was 170.1%, which was almost the same as in Comparative Example 4.

In contrast, in Example 40 in which both the water activity reducing agent and the emulsifier were used in the preparation of the lactic acid bacteria beverage, the acidity increase rate was significantly suppressed to 146.6%.

From these results, it is understood that the combination of an emulsifier and a water activity reducing agent can suppress the increase in acidity.

### [Test Example 4: Study of HLB of Emulsifier]

Tests were carried out similarly to Example 2 with the HLB of the used emulsifier (sucrose fatty acid ester) changed.

Skimmed milk powder and a water activity reducing agent (high fructose corn syrup) were dissolved in water, powdered lactic acid bacteria (FD-DVS *L.casei*-01) were added as a starter thereto, and the resultant was fermented at 37°C to obtain a fermented milk base.

To the thus obtained fermented milk base, any of various emulsifiers, water activity reducing agents (sugar and high fructose corn syrup), and a stabilizer (pectin) were added to be mixed so as to achieve a final composition shown in Table 23. The resultant mixture was filled into a plastic container and cooled overnight to obtain a lactic acid bacteria beverage. The lactic acid bacteria beverage thus obtained had a nonfat milk solid content of 3.43 wt%, and a protein content of 1.2 wt%. Table 23 shows the pHs of the lactic acid bacteria beverages thus obtained.

The followings were used as the emulsifier:
"Ryoto Sugar Ester S-570" (Mitsubishi Chemical Corporation, sucrose stearate, HLB = 5);
"Ryoto Sugar Ester S-770" (Mitsubishi Chemical Corporation, sucrose stearate, HLB = 7);
"Ryoto Sugar Ester S-970" (Mitsubishi Chemical Corporation, sucrose stearate, HLB = 9);
"Ryoto Sugar Ester P-1570" (Mitsubishi Chemical Corporation, sucrose palmitate, HLB = 15); and
"Ryoto Sugar Ester P-1670" (Mitsubishi Chemical Corporation, sucrose palmitate, HLB = 16).

### [Comparative Example 5]

A lactic acid bacteria beverage was obtained in the same manner as in Example 2 except that an emulsifier (sucrose fatty acid ester) was not added in the preparation of the lactic acid bacteria beverage. Table 23 shows the pH of the lactic acid bacteria beverage thus obtained.

Table 23 shows the results of the survival rate (i.e., the rate of the number of viable bacteria after 35 days storage to the number of viable bacteria after 0 days storage) calculated after storing the lactic acid bacteria beverage at 30°C for 29 days.

**[Table 23]**

| Entire Composition | Comparative Example 5 | Example 42 | Example 43 | Example 44 | Example 45 | Example 46 |
|---|---|---|---|---|---|---|
| Skimmed Milk Powder | 3.60% | 3.60% | 3.60% | 3.60% | 3.60% | 3.60% |
| Sucrose | 3.04% | 3.04% | 3.04% | 3.04% | 3.04% | 3.04% |
| High Fructose Corn Syrup | 17.60% | 17.60% | 17.60% | 17.60% | 17.60% | 17.60% |
| Sucrose Fatty Acid Ester (S-570) | 0% | 0.15% | 0% | 0% | 0% | 0% |
| Sucrose Fatty Acid Ester (S-770) | 0% | 0% | 0.15% | 0% | 0% | 0% |
| Sucrose Fatty Acid Ester (S-970) | 0% | 0% | 0% | 0.15% | 0% | 0% |
| Sucrose Fatty Acid Ester (P-1570) | 0% | 0% | 0% | 0% | 0.15% | 0% |
| Sucrose Fatty Acid Ester (P-1670) | 0% | 0% | 0% | 0% | 0% | 0.15% |
| Pectin | 0.25% | 0.25% | 0.25% | 0.25% | 0.25% | 0.25% |
| Water | 75.51% | 75.36% | 75.36% | 75.36% | 75.36% | 75.36% |
| Total | 100% | 100% | 100% | 100% | 100% | 100% |
| pH at Production | 3.63 | 3.65 | 3.65 | 3.65 | 3.64 | 3.64 |
| Survival Rate | 0.40% | 0.75% | 0.71% | 1.20% | 1.51% | 3.08% |

The survival rate was improved in all of Examples 42 to 46 compared to Comparative Example 5, and in particular, the improvement of the survival rate was remarkable in Examples 44 to 46 in which a sucrose fatty acid ester having an HLB of 9 or more was used.

### [Reference Test Example 1: Verification of Effect of Combined Use of Emulsifier and Water Activity Reducing Agent in Low Nonfat Milk Solid Food Product]

Three types of fermented milk bases were obtained by mixing milk (protein: 3.4 g/100 mL, lipid: 3.9 g/100 mL), skimmed milk powder (protein: 34 g/100 g, lipid: 0.13 g/100 g), fresh cream (protein: 1.7 g/100 mL, lipid: 47 g/100 mL), and water so as to achieve compositions shown in Table 24, and adding, to each of the resultant mixtures, Meiji Bulgaria Yogurt Plain (Meiji Co., Ltd.) in an amount of 2 wt% relative to the total amount of the fermented milk, and fermenting the resultant at 42°C for 3.5 hours. The fermented milk base having a composition 1 had a high nonfat milk solid content, the fermented milk base having a composition 2 had a low nonfat milk solid content, and the fermented milk base having a composition 3 was high in lipid (fats or oils).

**[Table 24]**

| Entire Composition | Composition 1 | Composition 2 | Composition 3 |
|---|---|---|---|
| Milk | 36.2% | 37.0% | 0.0% |
| Fresh Cream | 0.0% | 0.0% | 32.6% |
| Skimmed Milk Powder | 6.0% | 0.0% | 2.1% |
| Water | 55.7% | 60.9% | 63.2% |
| Starter (Bulgaria Yogurt) | 2.2% | 2.2% | 2.2% |
| Total | 100.0% | 100.0% | 100.0% |

To 92 parts by weight of each of the fermented milk bases (compositions 1 to 3), sucrose palmitate and water were added to be mixed so as to achieve a final composition shown in Table 25. The resultant mixtures were filled respectively into plastic containers and cooled overnight to obtain a fermented milk of Reference Example 47, and lactic acid bacteria beverages of Reference Examples 48 and 49. Table 25 shows the pHs and the numbers of viable bacteria in the fermented milk and the lactic acid bacteria beverages thus obtained.

Fermented milk and lactic acid bacteria beverages were obtained in the same manner as in Reference Examples 47 to 49 except that the emulsifier (sucrose fatty acid ester) was not added in the preparation of the fermented milk. Table 25 shows the pHs and the numbers of viable bacteria in fermented milk and lactic acid bacteria beverages thus obtained as Reference Comparative Examples 6 to 8.

After storing the fermented milk and the lactic acid bacteria beverages at 25°C for 14 days, the number of lactic acid bacteria was measured, and the acidity increase rate (i.e., the rate of the acidity after 14 days storage to the acidity after 0 days storage) was calculated. The results are shown in Table 25.

**[Table 25]**

| Entire Composition | Reference Comparative Example 6 | Reference Example 47 | Reference Comparative Example 7 | Reference Example 48 | Reference Comparative Example 8 | Reference Example 49 |
|---|---|---|---|---|---|---|
| Composition 1 | 92.00% | 92.00% | 0% | 0% | 0% | 0% |
| Composition 2 | 0% | 0% | 92.00% | 92.00% | 0% | 0% |
| Composition 3 | 0% | 0% | 0% | 0% | 92.00% | 92.00% |
| Sucrose Palmitate (added later) | 0% | 0.15% | 0% | 0.15% | 0% | 0.15% |
| Water (added later) | 8.00% | 7.85% | 8.00% | 7.85% | 8.00% | 7.85% |
| Total | 100.0% | 100.0% | 100.0% | 100.0% | 100.0% | 100.0% |
| Protein | 3.07% | 3.07% | 1.22% | 1.22% | 1.22% | 1.22% |
| Lipid | 1.39% | 1.39% | 1.36% | 1.36% | 14.19% | 14.19% |
| Carbonhydrate | 6.33% | 6.33% | 3.47% | 3.47% | 1.96% | 1.96% |
| pH at Production | 4.45 | 4.49 | 4.19 | 4.31 | 4.16 | 4.21 |
| Number of Viable Bacteria (cfu/ml) after storage for 0 day | 8.1E+08 | 7.7E+08 | 2.8E+08 | 2.8E+G7 | 3.2E+08 | 3.4E+08 |
| Acidity Increase Rate | 213.50% | 169.30% | - | - | 206.10% | 193.00% |

In Reference Example 47 (fermented milk), the number of viable bacteria immediately after the preparation (0 days storage) was comparable to that in Reference Comparative Example 6, in which an emulsifier was not added, and the increase in acidity after 14 days storage could be also suppressed. It is understood that when the nonfat milk solid content (such as protein) is high, a large number of viable bacteria can be obtained immediately after the preparation, and the increase in acidity during storage can also be suppressed, even without using a water activity reducing agent in combination.

On the other hand, in Reference Example 48 (lactic acid bacteria beverage), the number of viable bacteria immediately after the preparation (0 days storage) decreased to 1/10 compared to that in Reference Comparative Example 7 in which an emulsifier was not added. Therefore, a lactic acid bacteria beverage suitable for evaluating the suppression of post-fermentation (increase in acidity) during storage could not be obtained. When the nonfat milk solid content (such as protein) was low, the addition of an emulsifier alone led to a reduction in the number of viable bacteria immediately after the preparation, which suggests that a more advanced fermentation process control is required.

In Reference Example 49 (lactic acid bacteria beverage), the number of viable bacteria immediately after the preparation (0 days storage) was comparable to that in Reference Comparative Example 8 in which an emulsifier was not added, and the increase in acidity after 14 days storage could be mildly suppressed. It is understood that even when the nonfat milk solid content (such as protein) is low, if the content of lipid (fats or oils) is high, a certain degree of the effect, for the number of viable bacteria immediately after preparation and the suppression of the increase in acidity during storage, can be achieved by using only an emulsifier, without using a water activity reducing agent in combination. In other words, it is suggested that when both the nonfat milk solid content (such as protein) and the content of lipid (fats or oils) are low, particularly advanced control of the fermentation process is required to maintain the number of viable bacteria.

## Claims

1. A food product comprising a milk ingredient and a viable lactic acid bacterium, having a nonfat milk solid content of less than 8.0 wt%, a protein content of less than 2.7 wt%, and a pH of 4.6 or less, the food product comprising an emulsifier and a water activity reducing agent.

2. The food product according to claim 1, wherein a content of fats or oils is less than 10 wt%.

3. The food product according to claim 2, wherein the emulsifier has an HLB (Hydrophilic-Lipophilic Balance) of 9 or more.

4. The food product according to claim 3, wherein a content of the water activity reducing agent is 5 wt% or more.

5. The food product according to claim 1, wherein the emulsifier is one or more fatty acid esters selected from the group consisting of a sucrose fatty acid ester, a monoglyceride, an organic acid monoglyceride, a polyglycerol fatty acid ester, and a stearoyl lactylate.

6. The food product according to claim 5, wherein the emulsifier is a sucrose monofatty acid ester and/or a stearoyl lactylate, and is contained in an amount of 0.001 to 1.0 wt%.

7. The food product according to claim 1, wherein the water activity reducing agent is one or more selected from the group consisting of glucose, fructose, sucrose, erythritol, sorbitol, mannitol, isomaltooligosaccharide, xylooligosaccharide, oligotose, maltitol, palatinose, and xylitol.

8. The food product according to claim 7, having water activity Aw at 25°C of 0.990 or less.

9. The food product according to claim 8, having water activity Aw at 25°C of 0.981 or less.

10. The food product according to claim 1, comprising the viable lactic acid bacterium in an amount of 1 × 10² cfu/ml or more.

11. The food product according to claim 10, having a Y₁/X₁ value, calculated from an acidity increase rate X₁ (%) and a bacterial survival rate Y₁ (%) after storage at 25°C for 21 days from production, of 0.012 or more.

12. The food product according to claim 11, having a Y₂/X₂ value, calculated from an acidity increase rate X₂ (%) and a bacterial survival rate Y₂ (%) after storage at 30°C for 14 days from production, of 0.012 or more.

13. The food product according to claim 1, wherein the lactic acid bacterium is of the *Lactobacillus* genus (*Lactocaseibacillus* genus).

14. A method for producing a food product comprising a milk ingredient and a viable lactic acid bacterium, having a nonfat milk solid content of less than 8.0 wt%, a protein content of less than 2.7 wt%, and a pH of 4.6 or less, the method comprising:
(1) a step of adding a water activity reducing agent to the milk ingredient before fermentation by the lactic acid bacterium, and a step of adding an emulsifier to the milk ingredient after fermentation by the lactic acid bacterium;
(2) a step of adding a water activity reducing agent to the milk ingredient before fermentation by the lactic acid bacterium, and a step of adding an emulsifier and a water activity reducing agent to the milk ingredient after fermentation by the lactic acid bacterium; or
(3) a step of adding an emulsifier and a water activity reducing agent to the milk ingredient after fermentation by the lactic acid bacterium.

15. A method for suppressing increase in acidity and/or decrease in the number of viable bacteria in a food product comprising a milk ingredient and a viable lactic acid bacterium, having a nonfat milk solid content of less than 8.0 wt%, a protein content of less than 2.7 wt%, and a pH of 4.6 or less, the method comprising:
(1) a step of adding a water activity reducing agent to the milk ingredient before fermentation by the lactic acid bacterium, and a step of adding an emulsifier to the milk ingredient after fermentation by the lactic acid bacterium;
(2) a step of adding a water activity reducing agent to the milk ingredient before fermentation by the lactic acid bacterium, and a step of adding an emulsifier and a water activity reducing agent to the milk ingredient after fermentation by the lactic acid bacterium; or
(3) a step of adding an emulsifier and a water activity reducing agent to the milk ingredient after fermentation by the lactic acid bacterium.

16. Use of an emulsifier and a water activity reducing agent for suppressing increase in acidity and/or decrease in the number of viable bacteria, after production, in a food product comprising a milk ingredient and a viable lactic acid bacterium, having a nonfat milk solid content of less than 8.0 wt%, a protein content of less than 2.7 wt%, and a pH of 4.6 or less.

17. A food product comprising a milk ingredient and a viable lactic acid bacterium, having a nonfat milk solid content of less than 8.0 wt%, a protein content of less than 2.7 wt%, and a content of fats or oils of less than 10 wt%,
the food product comprising an emulsifier having an HLB of 9 or more, and
having water activity Aw at 25°C of 0.990 or less.

18. The food product according to claim 17, having water activity Aw at 25°C of 0.981 or less.

19. The food product according to claim 17, wherein the emulsifier is one or more fatty acid esters selected from the group consisting of a sucrose fatty acid ester, a monoglyceride, an organic acid monoglyceride, a polyglycerol fatty acid ester, and a stearoyl lactylate.

20. The food product according to claim 17, further comprising, as a water activity reducing agent, one or more selected from the group consisting of glucose, fructose, sucrose, erythritol, sorbitol, mannitol, isomaltooligosaccharide, xylooligosaccharide, oligotose, maltitol, palatinose, and xylitol.
